(19) Europäisches Patentamt
European Patent Office
Office européen des brevets

(11) EP 4 464 708 A1

(12) **EUROPEAN PATENT APPLICATION**

(43) Date of publication:
**20.11.2024 Bulletin 2024/47**

(21) Application number: **23174013.5**

(22) Date of filing: **17.05.2023**

(51) International Patent Classification (IPC):
***C07D 498/08*** *(2006.01)* ***A61K 49/10*** *(2006.01)*
***C07F 13/00*** *(2006.01)*

(52) Cooperative Patent Classification (CPC):
**C07D 498/08; A61K 49/106; C07F 15/025**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Bayer Aktiengesellschaft**
**51373 Leverkusen (DE)**

(72) Inventor: **The designation of the inventor has not yet been filed**

(74) Representative: **BIP Patents**
**c/o Bayer Intellectual Property GmbH**
**Alfred-Nobel-Straße 50**
**40789 Monheim am Rhein (DE)**

(54) **NEW CONTRAST AGENTS FOR USE IN DIAGNOSTIC IMAGING**

(57) The present invention relates to new manganese ($Mn^{2+}$) chelate compounds, to methods of preparing said compounds, to the use of said compounds as contrast agents in diagnostic imaging such as magnetic resonance imaging (MRI) and to their use in a mammalian body.

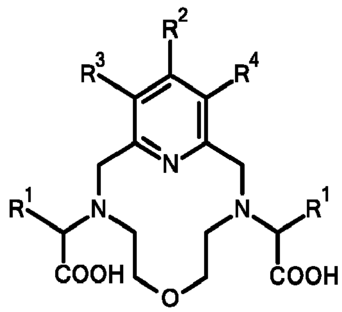

EP 4 464 708 A1

## Description

### FIELD OF THE INVENTION

The present invention relates to the items characterized in the patent claims, i.e. to new manganese ($Mn^{2+}$) chelate compounds, to methods of preparing said compounds, to the use of said compounds as contrast agents in diagnostic imaging such as magnetic resonance imaging (MRI) and to their use in a mammalian body.

### BACKGROUND

#### 1. Introduction

The Magnetic Resonance Imaging (MRI) technique is non-invasive and can provide information on the anatomy, function and metabolism of tissues in vivo. Unenhanced MRI scans of tissue anatomy and function make use of the hydrogen atoms in water to generate the image. Apart from differences in the local water content, the basic contrast in the MR image mainly results from regional differences in the intrinsic relaxation times T1 and T2, each of which can be chosen to dominate image contrast. However, the intrinsic contrast provided by the water T1 and T2 and changes in their values brought about by tissue pathology are often too limited to enable a sensitive and specific diagnosis. To overcome these limits the proton relaxation times can be influenced by the presence of paramagnetic ions. Commercial Gadolinium-based Contrast Agents (GBCAs) contain at least one paramagnetic ion of the rare earth metal Gadolinium ($Gd^{3+}$), which possesses the highest number of unpaired electrons of any stable ion (seven), creating a high magnetic moment that is effective at enhancing proton relaxation.

Paramagnetic contrast media shorten the T1 (longitudinal) and T2 (transversal) relaxation times of surrounding water protons to indirectly produce a signal-enhancing effect. The efficacy of an agent to shorten relaxation times is called relaxivity (r1 and r2), which is dependent on the ligand surrounding the paramagnetic ion and influenced by extrinsic factors including temperature, magnetic field strength and the matrix (water, solid tissue, or blood) (Lauffer RB et al., Paramagnetic metal complexes as water proton relaxation agents for NMR imaging: theory and design. Chem Rev. 1987;87(5):901-27; Caravan P et al., Gadolinium(III) chelates as MRI contrast agents: structure, dynamics, and applications. Chem Rev. 1999;99(9):2293-352).

Since unchelated $Gd^{3+}$ ions are toxic, extracellular GBCAs require ligands chelating the $Gd^{3+}$ ion. Gd-DTPA (gadopentetic acid, marketed as Magnevist, Bayer), Gd-DTPA-BMA (gadodiamide, marketed as Omniscan, Ge Healthcare) and Gd-BOPTA (gadobenate, marketed as MultiHance, Bracco) are classified as linear GBCAs based on the chain-like ligand structure. Gd-DOTA (gadoterate, e.g., marketed as Dotarem, Guerbet), Gd-HP-DO3A (gadoteridol, marketed as ProHance, Bracco) and Gd-DO3A-butrol (gadobutrol, e.g., marketed as Gadovist, Bayer) contain cage-like ligands and are classified as macrocyclic agents. Macrocyclic agents are more stable against dissociation and $Gd^{3+}$ release compared to linear agents (Frenzel et al. Invest Radiol. 2008 Dec;43(12):817-28.)

In 2014, a first study showed an increased signal intensity (SI) in the dentate nucleus (DN) and globus pallidus (GP) brain areas on unenhanced T1-weighted (T1w) MR images in patients with normal renal function who received multiple linear gadolinium-based contrast agent (GBCA) administrations (Kanda et al. Radiology 2014 Mar;270(3):834-41). This led to a thorough evaluation of GBCA safety including regulatory activities and intensive research on Gd retention in organs after use of GBCA. In March 2017, the European Union (EU) suspended the marketing authorization for all multi-purpose linear GBCAs while supporting the continued use of macrocyclic GBCAs.

Following this decision, the MRI market moved away from linear Gadolinium-based agents and increased efforts and strategies to reduce or replace Gd. Nonetheless, a new high relaxivity GBCA received market authorization in the US (gadopiclenol, marketed as Elucirem, Guerbet, and VUEWAY, Bracco) in 2022, which allows for a reduced dose while maintaining diagnostic efficacy. Other efforts include artificial intelligence approaches to further reduce gadolinium dose by creating virtual enhanced images comparable to full-dose images.

Thus, there is a long-standing desire to abandon GBCAs and to replace them with alternative contrast agents which remains unfulfilled to date. In particular, there is an increased long-standing medical need for new non-Gd contrast agents for magnetic resonance imaging. Endogenous paramagnetic elements have been considered as non-Gd alternatives for MRI. Manganese, one such endogenous paramagnetic element, is an essential trace element for several enzymes in the human body and possesses five unpaired electrons in its bivalent state.

To date, no multi-purpose contrast agent based on endogenous manganese is commercially available. Only one liver-specific Mn complex has been approved as a contrast agent for MRI: Mn-DPDP (mangafodipir, marketed as TeslaScan). Mn-DPDP (GE Healthcare) was approved for liver imaging at clinical doses of 0.005 mmol/kg but was withdrawn by the manufacturer from both the US and EU markets in 2003 and 2012, respectively). Mn-DPDP is metabolized in plasma and rapidly releases the Mn ions due to dephosphorylation and transmetalation with zinc. The released Mn-ions are cleared via biliary excretion resulting in the image contrast of the liver and detection of focal liver lesions

(Toft KG et al. Metabolism and pharmacokinetics of MnDPDP in man. Acta Radiol 1997 Jul;38(4 Pt 2):677-89.). Thus, the only non-Gd-based contrast agent approved so far was of limited practicability due to its liver specificity and was withdrawn from the market over 10 years ago. So far, no suitable alternative for intraveneous injection has been found.

Thus, there is an increased medical need to provide new contrast agents for radiological imaging. In particular, there is an increased medical need to provide new contrast agents based on endogenous elements replacing gadolinium. Given the low complex stability experienced with manganese (see Mn-DPDP above) and the clinically observed side effects, manganese-based contrast agents have been challenged as suitable and stable alternatives to multi-purpose GBCAs; instead, research and development efforts have focused on the use of other paramagnetic metal ions such as iron. Superparamagnetic iron oxide nanoparticles (SPIONS) have been investigated as MRI agents with very limited success: Two examples are Ferumoxytol (marketed as Feraheme and approved only for the treatment of iron deficiency and not for imaging purposes) and Ferucarbotran (marketed as Resovist, Bayer AG, discontinued in 2009). The pharmacokinetic clearance and biodistribution of SPIONS differ from those of GBCAs rendering them therefore not fully comparable (Jin et al. Superparamagnetic iron oxide nanoparticles for MR imaging and therapy: design considerations and clinical applications. Curr Opin Pharmacol. 2014 Oct;18:18-27). Iron chelates are alternatives that are still in a very early stage (Boehm-Sturm et al. Low-Molecular-Weight Iron Chelates May Be an Alternative to Gadolinium-based Contrast Agents for T1-weighted Contrast-enhanced MR Imaging; Radiology 2018 Feb;286(2):537. Snyder et al. A Class of FeIII Macrocyclic Complexes with Alcohol Donor Groups as Effective T1 MRI Contrast Agents. Angew Chem Int Ed Engl. 2020 Feb 3;59(6):2414-2419). Thus, there is a long-standing, increased medical need to provide Gd-free, multipurpose contrast agents.

## 2. Description of the prior art, problem to be solved and its solution

WO2021/043926 A1 describes Mn-based chelates, stereoisomers thereof and their properties including their potential use as contrast agents in MRI.

There is clearly an unmet medical need to provide new contrast agents for radiological imaging. particularly for magnetic resonance imaging which are not based on the use of Gadolinium.

The long-standing desire to abandon GBCAs and to replace them with alternative contrast agents remains unfulfilled to date. In particular, there is an increased long-standing medical need for new non-Gd contrast agents for magnetic resonance imaging. In addition, there is an increased long-standing medical need for new non-Gd contrast agents which applicability is not restricted to specific uses (such as liver imaging), i.e., multipurpose applications. Specifically, there is an unmet medical need to provide new contrast agents for radiological imaging which are not based on the use of Gadolinium and show as many of the below-listed criteria as possible:

- exhibit high water solubility,
- are physically and chemically stable,
- are sufficiently stable against metal release from the chelate,
- exhibit high relaxivity,
- have low protein binding,
- are fast and completely excreted,
- exhibit no long-term retention of Mn both in tissues and in organs,
- are stable against metabolic degradation,
- are well tolerated,
- can be produced in large quantities.

The state of the art described above does not disclose the compounds of general formula (I) of the present invention as defined herein, or a stereoisomer, a tautomer, an N-oxide, a hydrate, a solvate, or a salt thereof, or a mixture of the same, as described and defined herein. Nor does the art disclose the compounds of general formula (I) of the present invention in the form of a metal complex with $Mn^{2+}$, and stereoisomers, tautomers, N-oxides, hydrates, solvates, or salts thereof, or mixtures of same. Together or separately, the compounds of general formula (I) of the present

invention and the compounds of general formula (I) of the present invention in the form of a complex with $Mn^{2+}$, and stereoisomers, tautomers, N-oxides, hydrates, solvates, or salts thereof, or mixtures of same are hereinafter referred to as "compounds of the present invention". The compounds of general formula (I) of the present invention in the form of a complex with $Mn^{2+}$, and stereoisomers, tautomers, N-oxides, hydrates, solvates, or salts thereof, or mixtures of same may also be referred to as "$Mn^{2+}$-containing compounds of the present invention"

It has been found, and this constitutes the basis of the present invention, that the compounds of the present invention have surprising and advantageous properties.

In particular, the compounds of general formula (I) of the present invention allow for the preparation of complexes with $Mn^{2+}$, *i.e.* compounds of general formula (I) of the present invention in the form of a metal complex $Mn^{2+}$, respectively, as well as stereoisomers, tautomers, N-oxides, hydrates, solvates, or salts thereof, or mixtures of same. In particular, the $Mn^{2+}$-containing compounds of the present invention display the favorable stability of macrocyclic GBCAs without many of the drawbacks associated with the use of Gadolinium. The stability of the $Mn^{2+}$-containing compounds of the present invention can be determined by a number of physical and chemical tests, including, but not limited to, heat, oxidation, pH and light, among others.

Further, the $Mn^{2+}$-containing compounds of the present invention show high tolerability, sufficient relaxivity, excellent water solubility and a fast and complete excretion, making them well suited for diagnostic imaging, in particular for magnetic resonance imaging. The increased tolerability - as shown, for example, by cell toxicity assays - of the $Mn^{2+}$-containing compounds of the present invention is particularly advantageous, since toxicity has been a major concern for radiologists and patients. The ongoing evalution of GBCA safety has raised public awareness on potential toxicity and tolerability issues of contrast agents and has increased the demand for safer, more tolerable products.

## DESCRIPTION OF THE INVENTION

In accordance with a first aspect, the present invention covers compounds of general formula (I),

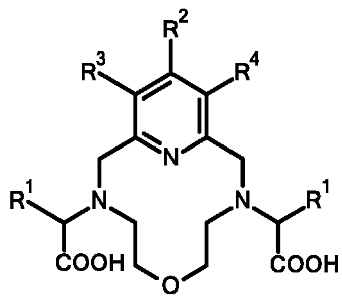

(I),

in which:

$R^1$ is a $-(CH_2)_m-(C=O)(NH)-(CH_2)-(CH(OH))_n-CH_2OH$ group
wherein m is an integer of from 1 to 3 and n is an integer of from 0 to 5;

$R^2$, $R^3$ and $R^4$ are each individually selected from a hydrogen atom, a $C_1$-$C_3$ alkyl group and a $C_1$-$C_3$ alkoxy group;

and stereoisomers, tautomers, N-oxides, hydrates, solvates, or salts thereof, or mixtures of same.

In accordance with a second aspect, the present invention covers compounds of general formula (I), *supra,* in the form of a complex with $Mn^{2+}$, and stereoisomers, tautomers, N-oxides, hydrates, solvates, or salts thereof, or mixtures of same.

As described in the present invention, compounds of general formula (I) in the form of a complex with $Mn^{2+}$, i.e., $Mn^{2+}$-containing compounds of the present invention refer to the compounds as depicted herein below:

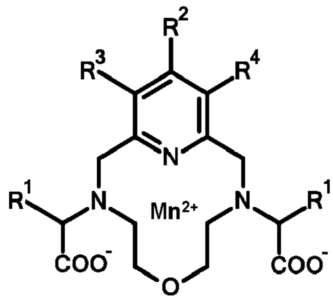

Thus, in accordance to said second aspect, the present invention covers compounds of general formula (I), *supra,* in the form of a complex with $Mn^{2+}$,
in which:

$R^1$ is a $-(CH_2)_m-(C=O)(NH)-(CH_2)-(CH(OH))_n-CH_2OH$ group,
wherein m is an integer of from 1 to 3 and n is an integer of from 0 to 5;

$R^2$, $R^3$ and $R^4$ are each individually selected from a hydrogen atom, a $C_1$-$C_3$ alkyl group and a $C_1$-$C_3$ alkoxy group;

and stereoisomers, tautomers, N-oxides, hydrates, solvates, or salts thereof, or mixtures of same.

**DEFINITIONS**

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero.

When groups in the compounds according to the invention are substituted, it is possible for said groups to be mono-substituted or poly-substituted with substituent(s), unless otherwise specified. Within the scope of the present invention, the meanings of all groups which occur repeatedly are independent from one another. It is possible that groups in the compounds according to the invention are substituted with one, two or three identical or different substituents, particularly with one substituent.

Should a composite substituent be composed of more than one parts, e.g. $(C_1$-$C_3$-alkoxy)-$(C_2$-$C_6$-alkyl)-, it is possible for the position of a given part to be at any suitable position of said composite substituent, *i.e.* the $C_1$-$C_3$-alkoxy part can be attached to any carbon atom of the $C_2$-$C_6$-alkyl part of said $(C_1$-$C_3$-alkoxy)-$(C_2$-$C_6$-alkyl)- group. A hyphen at the beginning or at the end of such a composite substituent indicates the point of attachment of said composite substituent to the rest of the molecule.

For example, in the context of the present invention, the compounds of formula (I) may comprise a group $R^1$ being a $-(CH_2)_m-(C=O)(NH)-(CH_2)-(CH(OH))_n-CH_2OH$ group, wherein m is an integer of from 1 to 3 and n is an integer of from 0 to 5. The hyphen at the beginning of the definition of $R^1$ indicates that this is the point of attachment of said group $R^1$ to the rest of the molecule, i.e., via the methylene "$(CH_2)_m$" group as shown below:

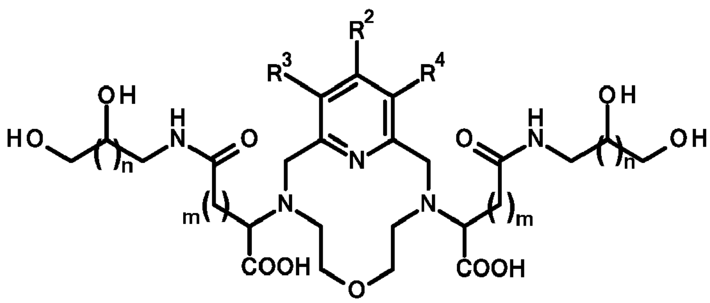

The term "comprising" when used in the specification includes "consisting of" and "consisting essentially of".

If within the present text any item is referred to as "as mentioned herein", it means that it may be mentioned anywhere in the present text.

The terms as mentioned in the present text have the following meanings:

The term "halogen atom" means a fluorine, chlorine, bromine or iodine atom, particularly a fluorine, chlorine or bromine atom.

The term "$C_1$-$C_6$-alkyl" means a linear or branched, saturated, monovalent hydrocarbon group having 1, 2, 3, 4, 5 or 6 carbon atoms, e.g. a methyl, ethyl, propyl, isopropyl, butyl, *sec*-butyl, isobutyl, *tert*-butyl, pentyl, isopentyl, 2-methylbutyl, 1-methylbutyl, 1-ethylpropyl, 1,2-dimethylpropyl, *neo*-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer or stereoisomer thereof. Particularly, said group has 1, 2, 3 or 4 carbon atoms ("$C_1$-$C_4$-alkyl"), *e.g.* a methyl, ethyl, propyl, isopropyl, butyl, sec-butyl isobutyl, or *tert*-butyl group, more particularly 1, 2 or 3 carbon atoms ("$C_1$-$C_3$-alkyl"), *e.g.* a methyl, ethyl, *n*-propyl or isopropyl group.

The term "$C_1$-$C_3$-haloalkyl" means a linear or branched, saturated, monovalent hydrocarbon group in which the term "$C_1$-$C_3$-alkyl" is as defined *supra,* and in which one or more of the hydrogen atoms are replaced, identically or differently, with a halogen atom. Particularly, said halogen atom is a fluorine atom. Said $C_1$-$C_3$-haloalkyl group is, for example, fluoromethyl, difluoromethyl, trifluoromethyl, 2-fluoroethyl, 2,2-difluoroethyl, 2,2,2-trifluoroethyl, pentafluoroethyl, 3,3,3-trifluoropropyl or 1,3-difluoropropan-2-yl or other polyfluorosubstituted alkyl group.

The term "$C_2$-$C_6$-hydroxyalkyl" means a linear or branched, saturated, monovalent hydrocarbon group in which the term "$C_2$-$C_6$-alkyl" is defined *supra,* and in which one or more, preferably 1, 2 or 3 of the hydrogen atoms are replaced with a hydroxy group, *e.g.* a 2-hydroxyethyl, 3-hydroxypropyl, 2-hydroxypropyl, 1-hydroxypropan-2-yl, 2,3-dihydroxypropyl, 1,3-dihydroxypropan-2-yl, 1,3-dihydroxy-2-(hydroxymethyl)propan-2-yl, 3-hydroxy-2-methyl-propyl, 2-hydroxy-2-methyl-propyl group.

The term "$C_1$-$C_3$-alkoxy" means a linear or branched, saturated, monovalent group of formula ($C_1$-$C_3$-alkyl)-O-, in which the term "$C_1$-$C_3$-alkyl" is as defined *supra, e.g.* a methoxy, ethoxy, *n*-propoxy or isopropoxy group.

The term "$C_3$-$C_6$-cycloalkyl" means a saturated, monovalent, mono- or bicyclic hydrocarbon ring which contains 3, 4, 5 or 6 carbon atoms ("$C_3$-$C_6$-cycloalkyl"). Said $C_3$-$C_6$-cycloalkyl group is for example, a monocyclic hydrocarbon ring, e.g. a cyclopropyl, cyclobutyl, cyclopentyl or cyclohexyl group.

The term "$C_1$-$C_6$", as used in the present text, e.g. in the context of the definition of "$C_1$-$C_6$-alkyl" means an alkyl group having a finite number of carbon atoms of 1 to 6, *i.e.* 1, 2, 3, 4, 5 or 6 carbon atoms.

Further, as used herein, the term "$C_3$-$C_6$", as used in the present text, *e.g.* in the context of the definition of "$C_3$-$C_6$-cycloalkyl", means a cycloalkyl group having a finite number of carbon atoms of 3 to 6, *i.e.* 3, 4, 5 or 6 carbon atoms.

When a range of values is given, said range encompasses each value and sub-range within said range.

For example:

"$C_1$-$C_6$" encompasses $C_1$, $C_2$, $C_3$, $C_4$, $C_5$, $C_6$, $C_1$-$C_6$, $C_1$-$C_5$, $C_1$-$C_4$, $C_1$-$C_3$, $C_1$-$C_2$, $C_2$-$C_6$, $C_2$-$C_6$, $C_2$-$C_4$, $C_2$-$C_3$, $C_3$-$C_6$, $C_3$-$C_5$, $C_3$-$C_4$, $C_4$-$C_6$, $C_4$-$C_5$, and $C_5$-$C_6$;

"$C_1$-$C_4$" encompasses $C_1$, $C_2$, $C_3$, $C_4$, $C_1$-$C_4$, $C_1$-$C_3$, $C_1$-$C_2$, $C_2$-$C_4$, $C_2$-$C_3$ and $C_3$-$C_4$;

"$C_1$-$C_3$" encompasses $C_1$, $C_2$, $C_3$, $C_1$-$C_3$, $C_1$-$C_2$ and $C_2$-$C_3$;

"$C_2$-$C_6$" encompasses $C_2$, $C_3$, $C_4$, $C_5$, $C_6$, $C_2$-$C_6$, $C_2$-$C_5$, $C_2$-$C_4$, $C_2$-$C_3$, $C_3$-$C_6$, $C_3$-$C_5$, $C_3$-$C_4$, $C_4$-$C_6$, $C_4$-$C_5$, and $C_5$-$C_6$; and

"$C_3$-$C_6$" encompasses $C_3$, $C_4$, $C_5$, $C_6$, $C_3$-$C_6$, $C_3$-$C_5$, $C_3$-$C_4$, $C_4$-$C_6$, $C_4$-$C_5$, and $C_5$-$C_6$.

The compounds of the present invention may contain one or more asymmetric centers, depending upon the location and nature of the various substituents desired. Asymmetric carbon atoms may be present in the (R) or (S) configuration, which can result in racemic mixtures, mixtures in which one enantiomer is present in a greater amount than the other enantiomer, or single enantiomers in the case of a single asymmetric center. In the case of multiple stereogenic centers, diastereomeric mixtures, single diastereomers or single enantiomers can be synthesized. In certain instances, asymmetry may also be present due to restricted rotation about a given bond, axial chirality or coordination of the metal center.

Preferred compounds are those which produce the more desirable biological activity. Separated, pure or partially purified isomers and stereoisomers or racemic or diastereomeric mixtures of the compounds of this invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or

chemical differences by methods known in the art, for example, by chromatography or fractional crystallisation. The optically active bases or acids are then liberated from the separated diastereomeric salts. A different process for separation of optical isomers involves the use of chiral chromatography (e.g., chiral HPLC columns), with or without conventional derivatisation, optimally chosen to maximise the separation of the enantiomers. Suitable chiral HPLC columns are manufactured by Daicel, e.g., Chiracel OD and Chiracel OJ among many others, all routinely selectable. Enzymatic separations, with or without derivatisation, are also useful. The optically active compounds of this invention can likewise be obtained by chiral syntheses utilizing optically active starting materials and/or reagents and catalysts.

In order to describe different types of isomers reference is made to IUPAC Rules Section E (Pure Appl Chem 45, 11-30, 1976).

The present invention includes all possible stereoisomers of the compounds of the present invention as single stereoisomers, or as any mixture of said stereoisomers, *e.g.* R- or S-isomers, or diastereoisomers, in any ratio. Isolation of a single stereoisomer, *e.g.* a single enantiomer or a single diastereomer, of a compound of the present invention may be achieved by any suitable state of the art method as described herein, such as chromatography, especially chiral chromatography, for example.

In particular, the compounds of general formula (I) and the compounds of general formula (I) in the form of a complex with $Mn^{2+}$, i.e. $Mn^{2+}$ complexes of the compounds of general formula (I), as described throughout this document and including their salts as described below, may exist in different stereochemical configurations (i.e., as different stereoisomers). The present invention therefore includes all possible stereoisomers of the compounds of general formula (I) in the form of a complex with $Mn^{2+}$, i.e. all stereoisomers of $Mn^{2+}$ complexes of the compounds of general formula (I) either as single stereoisomers or as mixtures of two or more of said stereoisomers, in any ratio.

In particular, the compounds of general formula (I) and the compounds of general formula (I) in the form of a complex with $Mn^{2+}$, i.e. $Mn^{2+}$ complexes of the compounds of general formula (I), as described throughout this document and including their salts as described below, exhibit chiral centres at the carbon atoms located alpha to the tertiary nitrogen atoms of the 12-membered macrocyclic ring, as denoted by * below:

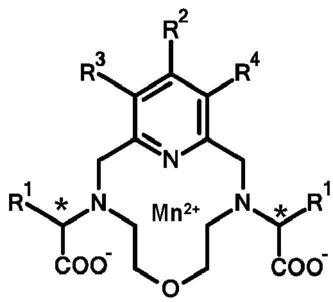

These carbon atoms can be in the (R) or (S) configuration. Thus, the present invention includes all compounds of general formula (I) and the compounds of general formula (I) in the form of a complex with $Mn^{2+}$, i.e. $Mn^{2+}$ complexes of the compounds of general formula (I), as described throughout this document and including their salts as described below, in which these carbon atoms are in the (R) or (S) configuration. Depending on their configuration, different diastereomers and/or enantiomers may exist. The present invention includes the RR, SS, RS and SR stereoisomers of the compounds of formula (I), and of the compounds of general formula (I) in the form of a complex with $Mn^{2+}$, i.e. $Mn^{2+}$ complexes of the compounds of general formula (I), as described throughout this document.

Given the chiral nature of the chelator, it is important that the stereochemical centers remain stable under the different chemical and/or physical conditions that they might be subjected to in the course of development, production, transport and/or administration of any drug product they might be contained in. In case a conformational change in any - i.e., one or more, if present - of the chiral centers should nonetheless occur, it is crucial that no diastereomer is form which has less desirable or even unfavorable properties. For example, it is important that no compounds - be it by-products or other diastereomers - with unfavorable or even harmful properties are formed during autoclaving - an important step in the preparation of sterile solutions of contrast agents - at high temperatures and/or high pressures.

Further, the compounds of the present invention can exist as N-oxides, which are defined in that at least one nitrogen of the compounds of the present invention is oxidized. The present invention includes all such possible N-oxides.

The present invention also relates to useful forms of the compounds as disclosed herein, such as hydrates, solvates, salts, in particular pharmaceutically acceptable salts, and co-precipitates.

The compounds of the present invention can exist as a hydrate, or as a solvate, wherein the compounds of the present invention contain polar solvents, in particular water, methanol or ethanol for example as structural element of the crystal lattice of the compounds. The amount of polar solvents, in particular water, may exist in a stoichiometric or non-stoichiometric ratio. In the case of stoichiometric solvates, e.g. a hydrate, hemi-, (semi-), mono-, sesqui-, di-, tri-,

tetra-, penta- *etc.* solvates or hydrates, respectively, are possible. The present invention includes all such hydrates or solvates.

Further, the compounds of the present invention may exist in the form of a salt depending on the nature of the substituents $R^1$, $R^2$, $R^3$ and $R^4$. Said salt may be either an inorganic or organic addition salt, particularly any pharmaceutically acceptable inorganic or organic addition salt, customarily used in pharmaceutical formulations.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19. The production of especially neutral salts is described in US Patent No. 5,560,903.

Pharmaceutically acceptable salts of the compounds according to the invention include salts with inorganic and/or organic bases or amino acids, in particular physiologically tolerable cations of inorganic and/or organic bases or amino acids, such as, inter alia, those of primary, secondary or tertiary amines. Examples may be, without being limited thereto, salts of sodium, lithium, potassium, calcium, magnesium, arginine, lysine, ammonia, creatinine, diethanolamine, ethanol amine, morpholine, glucamine, N,N-dimethylglucamine, N-methylglucamine, ornithine, histidine, imidazole, tromethamine, meglumine and the like. Particularly preferred pharmaceutically acceptable salts of the compounds according to the invention are their corresponding sodium salts.

Those skilled in the art will further recognize that salts of the claimed compounds may be prepared by reaction of the compounds with the appropriate inorganic or organic base via any of a number of known methods.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

This applies analogously to cases in which synthesis intermediates or example compounds or salts thereof have been obtained, by the preparation and/or purification processes described, as solvates, such as hydrates with (if defined) unknown stoichiometric composition.

**FURTHER EMBODIMENTS OF THE PRESENT INVENTION**

In accordance with a further embodiment of the first aspect, the present invention covers compounds of general formula (I), *supra,* wherein:

$R^1$ is a $-(CH_2)_m-(C{=}O)(NH)-(CH_2)-(CH(OH))_n-CH_2OH$ group
wherein m is an integer of from 1 to 2 and n is an integer of from 1 to 4;
$R^2$, $R^3$ and $R^4$ are each individually selected from a hydrogen atom, a $C_1$-$C_3$ alkyl group and a $C_1$-$C_3$ alkoxy group;
and stereoisomers, tautomers, N-oxides, hydrates, solvates, or salts thereof, or mixtures of same.

In accordance with a further embodiment of the first aspect, the present invention covers compounds of general formula (I), *supra,* wherein:

$R^1$ is a $-(CH_2)_m-(C{=}O)(NH)-(CH_2)-(CH(OH)_n-CH_2OH-$ group
wherein m is 2 and n is an integer of from 2 to 4;
$R^2$, $R^3$ and $R^4$ are each individually selected from a hydrogen atom, a $C_1$-$C_3$ alkyl group and a $C_1$-$C_3$ alkoxy group;
and stereoisomers, tautomers, N-oxides, hydrates, solvates, or salts thereof, or mixtures of same.

In accordance with a further embodiment of the first aspect, the present invention covers compounds of general formula (I), *supra,* wherein:

$R^1$ is a $-(CH_2)_m-(C{=}O)(NH)-(CH_2)-(CH(OH))_n-CH_2OH$ group
wherein m is 2 and n is an integer of from 2 to 4;
$R^2$, $R^3$ and $R^4$ are each individually a hydrogen atom;
and stereoisomers, tautomers, N-oxides, hydrates, solvates, or salts thereof, or mixtures of same.

In accordance with a further embodiment of the first aspect, the present invention covers compounds of general formula (I), *supra,* wherein:

$R^1$ is a $-(CH_2)_m-(C{=}O)(NH)-(CH_2)-(CH(OH))_n-CH_2OH$ group
wherein m is 2 and n is 4;

$R^2$, $R^3$ and $R^4$ are each individually a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Mn^{2+}$, wherein:

$R^1$ is a $-(CH_2)_m-(C=O)(NH)-(CH_2)-(CH(OH))_n-CH_2OH$ group
wherein m is an integer of from 1 to 2 and n is an integer of from 1 to 4;

$R^2$, $R^3$ and $R^4$ are each individually selected from a hydrogen atom, a $C_1$-$C_3$ alkyl group and a $C_1$-$C_3$ alkoxy group;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Mn^{2+}$, wherein:

$R^1$ is a $-(CH_2)_m-(C=O)(NH)-(CH_2)-(CH(OH))_n-CH_2OH$ group
wherein m is 2 and n is an integer of from 2 to 4;

$R^2$, $R^3$ and $R^4$ are each individually selected from a hydrogen atom, a $C_1$-$C_3$ alkyl group and a $C_1$-$C_3$ alkoxy group;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Mn^{2+}$, wherein:

$R^1$ is a $-(CH_2)_m-(C=O)(NH)-(CH_2)-(CH(OH))_n-CH_2OH$ group
wherein m is 2 and n is an integer of from 2 to 4;

$R^2$, $R^3$ and $R^4$ are each individually a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Mn^{2+}$, wherein:

$R^1$ is a $-(CH_2)_m-(C=O)(NH)-(CH_2)-(CH(OH))_n-CH_2OH$ group
wherein m is 2 and n is 4;

$R^2$, $R^3$ and $R^4$ are each individually a hydrogen atom;

and stereoisomers, tautomers, N-oxides, hydrates, solvates, or salts thereof, or mixtures of same.

In a further embodiment of the first aspect, the invention relates to compounds of formula (I), wherein:

$R^1$ is a $-(CH_2)_m-(C=O)(NH)-(CH_2)-(CH(OH))_n-CH_2OH$ group
wherein m is an integer of from 1 to 3 and n is an integer of from 1 to 4;

and stereoisomers, tautomers, N-oxides, hydrates, solvates, or salts thereof, or mixtures of same.

$R^1$ is a $-(CH_2)_m-(C=O)(NH)-(CH_2)-(CH(OH))_n-CH_2OH$ group
wherein m is an integer of from 1 to 2 and n is an integer of from 1 to 4;

and stereoisomers, tautomers, N-oxides, hydrates, solvates, or salts thereof, or mixtures of same.

In a further embodiment of the first aspect, the invention relates to compounds of formula (I), wherein:

$R^1$ is a $-(CH_2)_m-(C=O)(NH)-(CH_2)-(CH(OH))_n-CH_2OH$ group
wherein m is 2 and n is an integer of from 2 to 4;

and stereoisomers, tautomers, N-oxides, hydrates, solvates, or salts thereof, or mixtures of same.

In a further embodiment of the first aspect, the invention relates to compounds of formula (I), wherein:

$R^1$ is a $-(CH_2)_m-(C=O)(NH)-(CH_2)-(CH(OH))_n-CH_2OH$ group
wherein m is 2 and n is 4;

and stereoisomers, tautomers, N-oxides, hydrates, solvates, or salts thereof, or mixtures of same.

In a further embodiment of the first aspect, the invention relates to compounds of formula (I), wherein:

$R^2$, $R^3$ and $R^4$ are each individually selected from a hydrogen atom, a $C_1$-$C_3$ alkyl group and a $C_1$-$C_3$ alkoxy group;

and stereoisomers, tautomers, N-oxides, hydrates, solvates, or salts thereof, or mixtures of same.

In a further embodiment of the first aspect, the invention relates to compounds of formula (I), wherein:

$R^2$, $R^3$ and $R^4$ are each individually selected from a hydrogen atom and a $C_1$-$C_3$ alkyl group;

and stereoisomers, tautomers, N-oxides, hydrates, solvates, or salts thereof, or mixtures of same.

In a further embodiment of the first aspect, the invention relates to compounds of formula (I), wherein:

$R^2$, $R^3$ and $R^4$ are each individually a hydrogen atom;

and stereoisomers, tautomers, N-oxides, hydrates, solvates, or salts thereof, or mixtures of same.

It is to be understood that the present invention relates also to any combination of the embodiments described above.

Another embodiment of the first aspect is a compound of formula (I) which is 2-[9-(1,3-dicarboxypropyl)-6-oxa-3,9,15-triazabicyclo[9.3.1]pentadeca-1 (15), 11,13-trien-3-yl]pentanedioic acid and stereoisomers, tautomers, N-oxides, hydrates, solvates, or salts thereof, or mixtures of same.

In a further embodiment of the second aspect, the invention relates to compounds of formula (I) in the form of a complex with $Mn^{2+}$, wherein:

$R^1$ is a $-(CH_2)_m-(C=O)(NH)-(CH_2)-(CH(OH))_n-CH_2OH$ group
wherein m is an integer of from 1 to 3 and n is an integer of from 1 to 4;

and stereoisomers, tautomers, N-oxides, hydrates, solvates, or salts thereof, or mixtures of same.

$R^1$ is a $-(CH_2)_m-(C=O)(NH)-(CH_2)-(CH(OH))_n-CH_2OH$ group
wherein m is an integer of from 1 to 2 and n is an integer of from 1 to 4;

and stereoisomers, tautomers, N-oxides, hydrates, solvates, or salts thereof, or mixtures of same.

In a further embodiment of the second aspect, the invention relates to compounds of formula (I) in the form of a complex with $Mn^{2+}$, wherein:

$R^1$ is a $-(CH_2)_m-(C=O)(NH)-(CH_2)-(CH(OH))_n-CH_2OH$ group
wherein m is 2 and n is an integer of from 2 to 4;

and stereoisomers, tautomers, N-oxides, hydrates, solvates, or salts thereof, or mixtures of same.

In a further embodiment of the second aspect, the invention relates to compounds of formula (I) in the form of a complex with $Mn^{2+}$, wherein:

$R^1$ is a $-(CH_2)_m-(C=O)(NH)-(CH_2)-(CH(OH))_n-CH_2OH$ group
wherein m is 2 and n is 4;

and stereoisomers, tautomers, N-oxides, hydrates, solvates, or salts thereof, or mixtures of same.

In a further embodiment of the second aspect, the invention relates to compounds of formula (I) in the form of a complex with $Mn^{2+}$, wherein:

$R^2$, $R^3$ and $R^4$ are each individually selected from a hydrogen atom, a $C_1$-$C_3$ alkyl group and a $C_1$-$C_3$ alkoxy group;

and stereoisomers, tautomers, N-oxides, hydrates, solvates, or salts thereof, or mixtures of same.

In a further embodiment of the second aspect, the invention relates to compounds of formula (I) in the form of a complex with $Mn^{2+}$, wherein:

$R^2$, $R^3$ and $R^4$ are each individually selected from a hydrogen atom and a $C_1$-$C_3$ alkyl group;

and stereoisomers, tautomers, N-oxides, hydrates, solvates, or salts thereof, or mixtures of same.

In a further embodiment of the second aspect, the invention relates to compounds of formula (I) in the form of a complex with $Mn^{2+}$, wherein:

$R^2$, $R^3$ and $R^4$ are each individually a hydrogen atom;

and stereoisomers, tautomers, N-oxides, hydrates, solvates, or salts thereof, or mixtures of same.

It is to be understood that the present invention relates also to any combination of the embodiments described above.

Another embodiment of the second aspect are compounds of formula (I) in the form of a complex with $Mn^{2+}$, selected from the group consisting of: manganese(2+)4-carboxy-2-[9-(3-carboxy-1-carboxylatopropyl)-6-oxa-3,9,15-triazabicyclo[9.3.1]pentadeca-1(15),11,13-trien-3-yl}butanoate and manganese(2+) 2-[9-(1-carboxylato-4-oxo-4-{[(2S,3R,4R,5R)-2,3,4,5,6-pentahydroxyhexyl]amino}butyl)-6-oxa-3,9,15-triazabicyclo[9.3.1]pentadeca-1(15), 11,13-trien-3-yl]-5-oxo-5-{[(2S,3R,4R,5R)-2,3,4,5,6-pentahydroxyhexyl]amino}pentanoate and stereoisomers, tautomers, N-oxides, hydrates, solvates, or salts thereof, or mixtures of same.

In accordance with another aspect, the present invention covers methods of preparing compounds of the present invention, said methods comprising the steps as described in the Experimental Section herein.

In accordance with a further aspect, the present invention covers intermediate compounds which are useful for the preparation of the compounds of general formula (I), and particularly for the preparation of the compounds of general formula (I) in the form of a complex with $Mn^{2+}$, *supra.*

More particularly still, the present invention covers the intermediate compounds which are disclosed in the Experimental Section of this text, *infra.*

In particular, the compounds of formula (I) in the form of a complex with $Mn^{2+}$of the present invention can be used as contrast agents in contrast-enhanced MRI (CE-MRI), preferably for multi-purpose MRI.

A further aspect of the invention is the use of a compound of general formula (I), *supra,* for diagnostic imaging.

A person skilled in the art would recognize that the compounds of general formula (I) of this invention can be used in combination with other MR-active metal ions instead of $Mn^{2+}$, such compounds also being encompassed by the present invention.

A further aspect of the invention is the use of a compound of general formula (I), *supra,* in the form of a complex with $Mn^{2+}$ for diagnostic imaging.

A further aspect of the invention is the use of a $Mn^{2+}$ complex of a compound of general formula (I), *supra,* for diagnostic imaging.

Preferably, the use of a compound of the invention, *i.e.* of a compound of general formula (I), *supra* and/or of a compound of general formula (I), *supra,* in the form of a complex with $Mn^{2+}$ and/or of a $Mn^{2+}$ complex of a compound of general formula (I), *supra,* in the diagnosis is performed using magnetic resonance imaging (MRI).

Preferably, the use of a compound of the invention, *i.e.* of a compound of general formula (I), *supra* and/or of a compound of general formula (I), *supra,* in the form of a complex with $Mn^{2+}$ and/or of a $Mn^{2+}$ complex of a compound of general formula (I), *supra,* in the diagnosis is performed using magnetic resonance imaging (MRI).

A further aspect of the invention are compounds of general formula (I) for use in diagnostic imaging.

A further aspect of the invention are compounds of general formula (I) in the form of a complex with $Mn^{2+}$ for use in diagnostic imaging.

A further aspect of the invention are $Mn^{2+}$ complexes of compounds of general formula (I), *supra,* for use in diagnostic imaging.

A person skilled in the art would recognize that the compounds of general formula (I) of this invention can be used in combination with other MR-active metal ions instead of $Mn^{2+}$, such compounds also being encompassed by the present invention.

The invention also contains compounds of general formula (I) for the manufacture of diagnostic agents.

The invention also contains compounds of general formula (I) in the form of a complex with $Mn^{2+}$ for the manufacture of diagnostic agents.

A further aspect of the invention is the use of the compounds of general formula (I) or mixtures thereof for the manufacture of diagnostic agents.

A further aspect of the invention is the use of the compounds of general formula (I) in the form of a complex with $Mn^{2+}$ or mixtures thereof for the manufacture of diagnostic agents.

A further aspect of the invention is the use of the compounds of general formula (I) or mixtures thereof for the manufacture of diagnostic agents for magnetic resonance imaging (MRI).

A further aspect of the invention is the use of the compounds of general formula (I) in the form of a complex with $Mn^{2+}$ or mixtures thereof for the manufacture of diagnostic agents for magnetic resonance imaging (MRI).

A further aspect of the invention is a method of imaging body tissue in a patient, comprising the steps of administering to the patient an effective amount of one or more compounds of general formula (I) in the form of a complex with $Mn^{2+}$ in a pharmaceutically acceptable carrier, and subjecting the patient to NMR tomography.

A further aspect of the invention is a method of imaging body tissue in a patient, comprising the steps of administering to the patient an effective amount of one or more $Mn^{2+}$ complexes of the compounds of general formula (I) in a pharmaceutically acceptable carrier, and subjecting the patient to NMR tomography.

For the manufacture of diagnostic agents, for example the administration to human or animal subjects, the compounds of general formula (I) in the form of complexes with $Mn^{2+}$, *i.e.* $Mn^{2+}$ complexes of the compounds of general formula (I) or mixtures will conveniently be formulated together with pharmaceutical carriers or excipients. The contrast media of the invention may conveniently contain pharmaceutical formulation aids, for example stabilizers, antioxidants, pH-adjusting agents, metal scavengers, electrolytes (e.g. sodium chloride), flavors and the like. The diagnostic agents of the invention may be formulated for parenteral or enteral administration or for direct administration into body cavities. For example, in the case of $Mn^{2+}$ complexes of the compounds of general formula (I), parenteral formulations contain a sterile solution or suspension in a dose of 0.0001-5 mmol manganese/kg body weight, preferably 0.001-0.5 mmol manganese/kg body weight, more preferably 0.005-0.1 mmol manganese/kg body weight of the compound of formula (I) according to this invention. Thus, the media of the invention may be in conventional pharmaceutical formulations such as solutions, suspensions, dispersions, syrups, etc. in physiologically acceptable carrier media, preferably in water for injections. When the contrast medium is formulated for parenteral administration, it will be preferably isotonic or hypertonic and close to pH 7.4.

In a further aspect, the invention is directed to a method of diagnosing and health monitoring of patients. This method comprises a) administering to a human in need of such diagnosis a compound of the invention for detecting the compound in the human as described above and herein, and b) measuring the signal arising from the administration of the compound to the human, preferably by magnetic resonance imaging (MRI).

In a further aspect, the invention is directed to a method of diagnosing and health monitoring of patients. This method comprises a) administering to a human in need of such diagnosis a compound of the invention, *i.e.* a compound of general formula (I) in the form of a complex with $Mn^{2+}$ for detecting the compound in the human as described above and herein, and b) measuring the signal arising from the administration of the compound to the human, preferably by magnetic resonance imaging (MRI).

In a further aspect, the invention is directed to a method of diagnosing and health monitoring of patients. This method comprises a) administering to a human in need of such diagnosis a compound of the invention, *i.e.* a $Mn^{2+}$ complex of a compound of general formula (I) for detecting the compound in the human as described above and herein, and b) measuring the signal arising from the administration of the compound to the human, preferably by magnetic resonance imaging (MRI).

**GENERAL SYNTHESIS**

The compounds of general formula (I) described in this invention, including the compounds of general formula (I) in the form of a complex with $Mn^{2+}$ can be produced using the following general procedure depicted in Scheme 1 below. Unless otherwise specified, the groups $R^1$ to $R^4$ and m displayed therein have the meaning given in the description above. In case protective group chemistry is required for the introduction of YCHCOOH, the group $R^5$ can be employed.

Thus, should no protective group chemistry be needed, the group $R^5$ equals H, In general, but not exclusively, $R^5$ is selected from methyl, ethyl or tert-butyl.

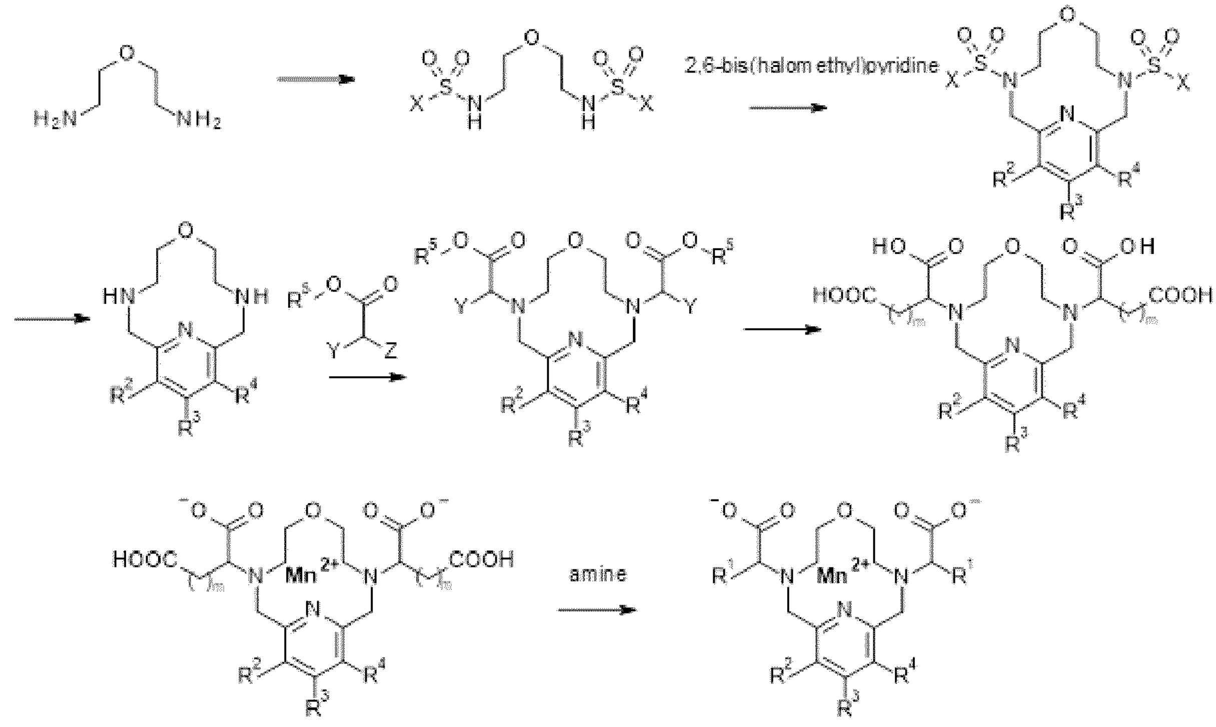


## Scheme 1

For example the commercially available bis-(2-aminoethyl)-ether can be tosylated or nosylated with the corresponding bromides or chlorides in the presence of base like potassium carbonate or sodium carbonate. Cyclization with an activated pyridine such as 2,6-bis(chloromethyl)pyridine (CAS 3099-28-3) or 2,6-bis(bromoomethyl)pyridine (CAS 7703-74-4) can be carried out in the presence of base like potassium carbonate or sodium carbonate. De-tosylation using concentrated sulphuric acid or treatment of the bis-nosylate with benzenethiol and base can deliver the free diamine. Alkylation with $\alpha$-halo- or $\alpha$-sulphonyl- bis-carboxylic acid esters or nitriles can be carried out in the presence of base like potassium carbonate or sodium carbonate. After deprotection, complexation using $MnCl_2$ can be carried out in aqueous solution at 50°C to 110°C in a pH range from 5 to 12. Activation on non-Manganese bound carboxylates with peptide reagents like EDCI and/or HOBT can be used to couple with suitable amines to result in the desired compound of Formula I. Literature for the synthetic procedures shown in scheme 1 can be found (see for example WO 2017/089849, WO 2019/122255, J. Henig, E. Toth, J. Engelmann, S, Gottschalk, ,H. Mayer, H. Inorganic Chemistry 2010, 49(13), 6124-38, EP2457914 B1.)

The scheme and procedure described above illustrate synthetic route to the compounds of general formula (I) of the invention and are not intended to be limiting. It would be apparent to the person skilled in the art that the order of transformations as exemplified in the scheme can be modified in various ways. The order of transformations exemplified in the schemes is therefore not intended to be limiting. Appropriate protecting groups and their introduction and cleavage are well-known to the person skilled in the art (see for example T.W. Greene and P.G.M. Wuts in Protective Groups in Organic Synthesis, 3rd edition, Wiley 1999). Specific examples are described in the subsequent paragraphs.

## DESCRIPTION OF THE FIGURES

Figure 1. Complex stability of Mn chelate example 2 after addition of $ZnCl_2$ using 18 equivalents of $Zn^{2+}$. Percent of intact Mn chelate example 2 measured over a time period of 7 days. Measurements were performed in 100mM BIS-TRIS at pH 7.

Figure 2. Complex stability of Mn chelate example 2 in human plasma. Percent of intact Mn chelate example 2

measured over a time period of 24h. The test item was investigated at 100μmol/L concentration in human plasma containing heparin as an anti coagulant.

Figure 3. In vitro cell toxicity in SH-SY5Y cells. Percent of viable cells after incubation with Mn chelate example 2 and Mn chelate-5 (WO2021/043926). All compounds were tested at 1 mmol/L and 5 mmol/L for 24h.

## EXPERIMENTAL SECTION

Chemical names were generated using the ACD/Name software from ACD/Labs. In some cases generally accepted names of commercially available reagents were used in place of ACD/Name generated names.

The following table 1 lists the abbreviations used in this paragraph and in the Examples section as far as they are not explained within the text body. Other abbreviations have their meanings customary *per se* to the skilled person.

**Table 1:** Abbreviations

| **Abbreviation** | **Meaning** |
|---|---|
| AcOH | acetic acid |
| ACN | acetonitrile |
| aq. | aqueous |
| AUC | Area under the curve |
| br | broad signal (NMR) |
| bw | Body weight |
| $C_{Gd}$ | Gadolinium concentration |
| CPMG | Carr-Purcell-Meiboom-Gill (MRI sequence) |
| d | doublet (NMR) |
| DAD | Diode Array Detector |
| DAST | Diethylaminosulfur trifluoride |
| DBU | 1,8-diazabicyclo(5.4.0)undec-7-ene |
| DCM | Dichloromethane |
| dd | doublet of doublet (NMR) |
| DIPEA | diisopropylethylamine |
| DMA | *N*,*N*-dimethylacetamide |
| DMAP | 4-dimethylaminopyridine |
| DMF | *N*,*N*-dimethylformamide |
| DMSO | Dimethylsulfoxide |
| EDC.HCl | N-(3-Dimethylaminopropyl)-N'-ethylcarbodiimide hydrochloride salt |
| ESI | electrospray (ES) ionization |
| EtOAc | ethyl acetate |
| EtOH | ethanol |
| FLASH | Fast Low Angle Shot |
| Gd | Gadolinium |
| h | hour(s) |
| HATU | Hexafluorophosphate azabenzotriazole tetramethyl uronium |
| HCl | hydrogen chloride, hydrochloric acid |
| HPLC | high performance liquid chromatography |

(continued)

| Abbreviation | Meaning |
|---|---|
| ICP-MS | inductively coupled plasma mass spectrometry |
| IR | Inversion Recovery |
| LC-MS | liquid chromatography-mass spectrometry |
| m | multiplet (NMR) |
| mCPBA | meta-Chloroperoxybenzoic acid |
| MeCN | Acetonitrile |
| MeOH | Methanol |
| min | minute(s) |
| MS | mass spectrometry |
| MTBE | Methyl-*tert*-butylether |
| MRI | Magnetic Resonance Imaging |
| MWD | multiple wavelength detector |
| NaCL | sodium chloride |
| NMR | Nuclear Magnetic Resonance spectroscopy : chemical shifts ($\delta$) are given in ppm. The chemical shifts were corrected by setting the DMSO signal to 2.50 ppm using unless otherwise stated. |
| q | quartet (NMR) |
| $r_i$ | relaxivities (where i=1, 2) |
| $r_1$ or r1 | reflect how T1 relaxation rates change as a function of contrast agent concentration (slope T1 versus Gd concentration) |
| $r_2$ or r2 | reflect how T2 relaxation rates change as a function of contrast agent concentration (slope T2 versus Gd concentration) |
| $R_1$ | longitudinal relaxation rate ($1/r_1$) |
| $R_2$ | transversal relaxation rate ($1/ r_2$) |
| Rt or RT | room temperature |
| $R_t$, Rt | retention time |
| s | singulet (NMR) |
| sat. | saturated |
| SFC | supercritical fluid chromatography |
| $T_1$ or T1 | longitudinal relaxation time (T1 relaxation time) |
| $T_2$ or T2 | transversal relaxation time (T2 relaxation time) |
| t | triplet (NMR) |
| td | triplet of doublet (NMR) |
| TEA | triethylamine |
| TFA | trifluoroacetic acid |
| THF | tetrahydrofuran |
| $\delta$ | chemical shift |

Other abbreviations have their meanings customary *per se* to the skilled person.

The various aspects of the invention described in this application are illustrated by the following examples which

are not meant to limit the invention in any way.

The example testing experiments described herein serve to illustrate the present invention and the invention is not limited to the examples given.

## EXPERIMENTAL SECTION - GENERAL PART

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be removed by trituration using a suitable solvent. In some cases, the compounds may be purified by chromatography, particularly flash column chromatography, using for example prepacked silica gel cartridges, e.g. Biotage SNAP cartridges KP-Sil® or KP-NH® in combination with a Biotage autopurifier system (SP4® or Isolera Four®) and eluents such as gradients of hexane/ethyl acetate or DCM/methanol. In flash column chromatography, unmodified ("regular") silica gel may be used as well as aminophase functionalized silica gel. If reference is made to flash column chromatography or to flash chromatography in the experimental section without specification of a stationary phase, regular silica gel was used.

In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

### Analytical LC-MS Methods:

Method **1**

Instrument: Waters Acquity UPLC-MS SQD 3001; Column: Acquity UPLC BEH C18 1.7 μm, 50 x 2.1 mm; eluent A: water + 0.2 vol % ammonia, Eluent B: acetonitrile; Gradient: 0-1.6 min 1-99% B, 1.6-2.0 min 99% B; Flow rate: 0.8 mL/min; Temperature: 60 °C; Injection: 2 μL; DAD scan: 210-400 nm; ELSD.

Method **2**

Instrument: Waters Acquity UPLC-MS SQD 3001; Column: Acquity UPLC BEH C18 1.7 μm, 50 x 2.1 mm; eluent A: water + 0.2 vol% ammonia, Eluent B: acetonitrile; Gradient: 0-1.6 min 1-99% B, 1.6-2.0 min 99% B; Flow rate: 0.8 mL/min; Temperature: 60 °C; Injection: 2 μL; DAD scan: 210-400 nm; ELSD.

Method **3**

Instrument : SHIMADZU LCMS-2020; column: Kinetex EVO C18, 5 μm, 30 x 2.1 mm; mobile phase A: water + 0.0375 vol% TFA, B: acetonitrile + 0.01875 vol% TFA; gradient: 0.01 min 5.00% B→ 0.80 min 95.0% B→ 1.20 min 95.0% B→ 1.21 min 5.00% B→ 1.55 min 5.00% B; flow rate: 1.5 mL/min; Column temperature: 50 °C; UV detection: PAD(220 nm & 254 nm).

### NMR Spectra

The multiplicities of proton signals in $^1H$ NMR spectra given in the following paragraphs reflect the observed signal form and do not take into account any higher-order signal phenomena. As a rule, the chemical shift data refers to the center of the signal in question. In the case of wide multiplets, a range is specified. Signals hidden by solvent or water were either assigned tentatively or are not listed. Strongly broadened signals - *e.g.* caused by rapid rotation of

molecular moieties or by interchanging protons - have also been assigned tentatively (often referred to as a broad multiplet or broad singlet) or are not shown.

The $^1$H-NMR data of selected compounds are listed in the form of $^1$H-NMR peaklists. Therein, for each signal peak the $\delta$ value in ppm is given, followed by the signal intensity, reported in round brackets. The $\delta$ value-signal intensity pairs from different peaks are separated by commas. Therefore, a peaklist is described by the general form: $\delta_1$ (intensity,), $\delta_2$ (intense), ... , $\delta_i$ (intensity$_i$), ... , $\delta_n$ (intensity$_n$).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A $^1$H-NMR peaklist is similar to a classical $^1$H-NMR readout, and thus usually contains all the peaks listed in a classical NMR interpretation. Moreover, similar to classical $^1$H-NMR printouts, peaklists can show solvent signals, signals derived from stereoisomers of the particular target compound, peaks of impurities, $^{13}$C satellite peaks, and/or spinning sidebands. The peaks of stereoisomers, and/or peaks of impurities are typically displayed with a lower intensity compared to the peaks of the target compound (*e.g.*, with a purity of >90%). Such stereoisomers and/or impurities may be typical for the particular manufacturing process, and therefore their peaks may help to identify a reproduction of the manufacturing process on the basis of "by-product fingerprints". An expert who calculates the peaks of the target compound by known methods (MestReC, ACD simulation, or by use of empirically evaluated expectation values), can isolate the peaks of the target compound as required, optionally using additional intensity filters. Such an operation would be similar to peak-picking in classical $^1$H-NMR interpretation. A detailed description of the reporting of NMR data in the form of peaklists can be found in the publication "Citation of NMR Peaklist Data within Patent Applications" (cf. http://www.researchdisclosure.com/searching-disclosures, Research Disclosure Database Number 605005, 2014, 01 Aug 2014). In the peak picking routine, as described in the Research Disclosure Database Number 605005, the parameter "MinimumHeight" can be adjusted between 1% and 4%. However, depending on the chemical structure and/or depending on the concentration of the measured compound it may be reasonable to set the parameter "MinimumHeight" <1%.

**Synthesis of the Intermediates**

**intermediate 1**

**dimethyl 2-[9-(4-methoxy-1-methoxycarbonyl-4-oxo-butyl)-6-oxa-3,9,15-triazabicyclo[9.3.1]pentadeca-1(14),11(15),12-trien-3-yl]pentanedioate**

To a solution of 6-oxa-3,9,15-triazabicyclo[9.3.1]pentadeca-1(15),11,13-triene (CAS RN 2098905-67-8 as 3HBr salt, 30.0 g, 66.7 mmol) in MeCN (600 mL) was added potassium carbonate (55.3 g, 400 mmol) and dimethyl 2-bromopentanedioate (33.5 g, 140 mmol). The mixture was stirred at 25 °C for 12 hrs. The reaction mixture was filtered and the filtrate concentrated under reduced pressure to give a residue. The residue was purified by column chromatography (silica gel, dichloromethane/methanol 10/1) to give 21 g, of the crude title compound as a yellow oil. Further purification by preparative HPLC (column: Phenomenex Luna C18 (250 × 80 mm, 15 μm); mobile phase: [water (TFA)-MeCN]; B%: 15%-45%, 20 min), yielded the title compound as its TFA salt (18.0 g, 17.3 mmol, 33.2% yield, 83.0% purity, 3TFA) as a yellow oil confirmed by LCMS (EW34579-37-p1a3, Rt = 0.827 min), HPLC (EW34579-37-p1b3, Rt = 2.566 min), HNMR (EW34579-37-p1).

LCMS (Method 3): Rt = 0.631 min, m/z = 524.3(M+H$^+$)

$^1$H NMR: (400 MHz, $CDCl_3$) $\delta$ 8.39-8.29 (m, 1H), 7.69-7.27 (m, 2H), 4.48-4.39 (m, 4H), 3.73-3.69 (m, 6H), 3.68-3.63 (m, 2H), 3.63-3.62 (m, 6H), 3.48-3.42 (m, 4H), 3.03-2.99 (m, 4H), 2.51-2.46 (m, 4H), 2.14-2.12 (m, 2H) , 2.10-1.95 (m, 2H) ppm.

**intermediate 2**

**2-[9-(1,3-dicarboxypropyl)-6-oxa-3,9,15-triazabicyclo[9.3.1]pentadeca-1 (15),11,13-trien-3-yl]pentanedioic acid**

A solution of dimethyl 2-[9-(4-methoxy-1-methoxycarbonyl-4-oxo-butyl)-6-oxa-3,9,15-triazabicyclo[9.3.1]pentadeca-1(14),11(15),12-trien-3-yl]pentanedioate (Intermediate 1, 18.0 g, 20.8 mmol, 3TFA) in aqueous HCl (6.00 M, 180 mL) was stirred at 80°C for 5 hours. The reaction mixture was lyophilized to give a yellow solid. The residue was purified by prep-HPLC (column: Phenomenex Luna C18, 10 μm, 250 x 80 mm; mobile phase: [water (HCl)-MeCN]; B%: 0%-20%, 20min.), to give 8.7 g of the title compound as HCl salt (72% yield, 99% purity).

LC-MS (method 3): $R_t$ = 0.49 min; MS (ESIpos): m/z = 468.2 $[M+H]^+$.

$^1H$ NMR: (400 MHz, $D_2O$) δ 8.05-8.00 (m, 1H), 7.52-7.49 (m, 2H), 4.87-4.75 (m, 2H), 4.20-4.14 (m, 2H), 3.62-3.41 (m, 8H), 2.75-2.68 (m, 4H), 2.29-2.24 (m, 4H) ppm.

## Synthesis of the Examples

### Example 1

**manganese($^{2+}$) 4-carboxy-2-[9-(3-carboxy-1-carboxylatopropyl)-6-oxa-3,9,15-triazabicyclo[9.3.1]pentadeca-1(15),11,13-trien-3-yl}butanoate**

The pH of 2-{9-[-1,3-dicarboxypropyl]-6-oxa-3,9,15-triazabicyclo[9.3.1]pentadeca-1(15),11,13-trien-3-yl}pentanedioic acid hydrogen chloride (1/1) (4.00 g, 7.94 mmol) in water (40 mL) was adjusted to pH 7 by addition of aqueous ammonium hydroxide (33%). Manganese(II) chloride tetrahydrate (1.57 g, 7.94 mmol) was added and the mixture was stirred 10 hours at 100°C and one hour at 120°C. Chelex® 100 was added to the cooled reaction solution and the mixture was stirred for 120 minutes. The crude product was purified by column chromatography (Biotage®Sfär C18 D, water / acetonitrile) to give 4.0 g of the title compound as a mixture of stereoisomers (95% purity, 61% yield).

LC-MS (method 2): $R_t$ = 0.48 min and 0.54 min; MS (ESIpos): m/z = 521 $[M+H]^+$

### Example 2

**manganese($^{2+}$) 2-[9-(1-carboxylato-4-oxo-4-{[(2*S*,3*R*,4*R*,5*R*)-2,3,4,5,6-pentahydroxyhexyl]amino}butyl)-6-oxa-3,9,15-triazabicyclo[9.3.1]pentadeca-1(15),11,13-trien-3-yl]-5-oxo-5-{[(2S,3R,4R,5R)-2,3,4,5,6-pentahydroxyhexyl]amino}pentanoate**

The pH of 1-amino-1-deoxy-D-glucitol (CAS-RN:488-43-7, 1.16 g, 6.43 mmol) in water (14 mL) was adjusted to pH 7 by addition of aqueous hydrochloric acid (1M). Manganese($^{2+}$) 4-carboxy-2-[9-(3-carboxy-1-carboxylatopropyl)-6-oxa-3,9,15-triaza bicyclo[9.3.1]pentadeca-1(15),11, 13-trien-3-yl}butanoate (Example 1, 700 mg, 1.35 mmol), 1-(3-dimethylaminopropyl)-3-ethylcarbodiimide hydrochloride (CAS-RN: 25952-53-8, 609 mg, 3.17 mmol) and 1-hydroxybenzotriazole hydrate (CAS-RN: 123333-53-9, 86.5 mg, 565 μmol) were added and the mixture was stirred for 16 hours at room temperature. Additional 1-amino-1-deoxy-D-glucitol (260 mg, 1.43 mmol) and 1-(3-dimethylaminopropyl)-3-ethylcarbodiimide hydrochloride (304 mg, 1.59 mmol) were added and the pH was adjusted to pH 6 by addition of aqueous hydrochloric acid (1M). After stirring for 16 hours at room temperature the mixture was heated to 120°C for 2 hours. After cooling to room temperature, the mixture was concentrated under reduced pressure. The residue was purified by column chromatography (Biotage®Sfär C18 D, water / acetonitrile) to give 802 mg of the crude title compound The crude product was purified by preparative HPLC (Instrument: Labomatic HD-5000, pump head HDK-280, gradient module NDB-1000, manual injection valve Rheodyne 3725i038, fraction collector: Labomatic Labocol Vario 2000, Knauer UV detector Azura UVD 2.1S, Prepcon 5 software; column: Chromatorex RP C18 10μm, 120 x 30 mm; eluent A: water ; eluent B: acetonitrile; gradient: 0-10 min 1-3% B, 10-12 min 99% B; Flow rate: 100 mL/min; Temperature: 25 °C) to yield 289 mg of the title compound as a mixture of stereoisomers (95% purity, 24% yield).

LC-MS (method 1): $R_t$ = 0.40 min; MS (ESIpos): m/z = 847 $[M+H]^+$, 424$[M+2H]^{2+}$

## EXPERIMENTAL SECTION - PHYSCOCHEMICAL AND BIOLOGICAL ASSAYS

### In vitro and in vivo characterization of example compounds

Examples were tested in selected assays one or more times. When tested more than once, data are reported as either average values or as median values, wherein

- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from assays represent average values or median values calculated utilizing data sets obtained from testing of one or more synthetic batch.

**Example A - Water solubility**

The exploratory water solubility of the compounds is determined at room temperature (20°C) in buffer solution (10 mM Tris-HCl, pH 7.4). The solid compounds were added stepwise to the buffer solution. More substance is added once all the suspended material is dissolved until an equilibrium between undissolved and dissolved substance is reached. The suspension was mixed using a shaker (Heidolph Reax 2000) and treated 5 min in an ultrasound bath (Bandelin, Sonorex Super RK255H). The Manganese concentration of the test substance in the clear solution is measured by ICP-MS.

**Example B - Relaxivity measurements at 1.4 T**

T1 and T2 relaxation time were measured at 60 MHz (1.41 T) and 37°C using a Bruker mq60 minispec contrast agent analyzer. Manganese containing chelates were dissolved in different media at and each relaxivity measurement was performed using three different Mn concentrations. Measurement of r1 and r2 relaxivities were performed using concentrations of 0, 0.25, 0.5 and 1mM Mn in water and human plasma containing heparin as anticoagulant. The relaxivities $r_i$ (where i=1, 2) were calculated based on the measured relaxation rates $R_i$ in water and plasma:

$$r_i = (R_i - R_{i(0)}) / C_{Mn}$$

where $R_{i(0)}$ represent the relaxation rate of the respective solvent and $C_{Mn}$ the concentration of the compound normalized to Manganese. The Manganese concentrations of the investigated solutions were verified by Inductively Coupled Plasma Mass Spectrometry (ICP-MS Agilent 7500a, Waldbronn, Germany). The determined relaxivity values are summarized in Table 1.

Table 1: Relaxivities of investigated compound in water and human plasma at 1.41 T. All values are measured at 37°C and normalized to Mn and given in L mmol-1 s-1.

| | | Mn chelate example 2 |
|---|---|---|
| r1 (L $mM^{-1}s^{-1}$) | water | 5.0 |
| | human plasma | 6.4 |
| R2 (L $mM^{-1}s^{-1}$) | water | 16.3 |
| | human plasma | 19.6 |

**Example C - Chemical stability (PhysChem)**

Examples were separately dissolved in appropriate media/buffer.

The stability of the Mn-chelate was tested using different conditions:

a) Heat: The Mn chelate was dissolved in 10 mM Tris-HCl buffer, pH 7.4 at a final concentration of 1 mmol Mn/L. The solution was autoclaved three times at 1 bar, 121°C for 20 min.

b) Oxidation: Assessment of stability of the test item after addition of oxidants such as $H_2O_2$ and O2.

c) pH: Assessment of stability of the test item in solutions at different pH values which can be performed at different temperatures, for example at 25°C and 121°C.

d) Light: Assessment of stability after exposure of the test item to light.

Aliquots of the Mn-chelate during the tests were removed, frozen at -20°C for later analytical analyses by HPLC-ICP-MS or HPLC-ESI-MS to determine the integrity of the compound.

Examples of used HPLC methods: Agilent 1290 Infinity II LC, Agilent ICP-MS 7900, Column: Waters BEH Acquity C18 UPLC, 1.7 μm, 2.1 × 50 mm. Solvent A: 50 mmol/L NH4HCO3 pH 8.0. Solvent B: MeOH. Gradient from 0% B to 0.7% B within 6.5 min, flow 0.8 mUmin.Detection by ICP-MS or ESI-MS. The chromatograms, displaying the intensity of the detected Mn were compared.

The chemical stability of isomers and isomer conversion were compared.

**Example D - Complex stability (Transmetallation)**

The stability of the complex against other metals, for example Zn, was assessed using relaxivty as a readout. Mn chelates were dissolved in 100 mM BIS-TRIS Buffer at pH 7. T2 relaxation rates were measured at 37°C using a Bruker mq60 minispec contrast agent analyzer. T2 relaxation rates were periodically monitored after addition of a ZnCl2 solution resulting in a Mn:Zn ratio of 1:18. If test compounds are instable under these conditions, transmetallation during this test would lead to the formation of the $Zn^{2+}$ complex of the test item, while $Mn^{2+}$ is released and present in the solution as $[Mn(H_2O)_6]^{2+}$. Since $[Mn(H_2O)_6]^2$ influences the T2 relaxation time, this ongoing exchange reaction would lead to decreasing T2 relaxation times of the solution over time (measured at 1.41 T).

The stability of the Mn chelate was determined based on the measurements of the relaxation rate of the Mn-chelate and calculated with the known Mn concentration in the assay and the known relaxivity of $[Mn(H_2O)_6]^{2+}$ (r2 = 57 $mM^{-1}s^{-1}$) and the known relaxation rate of the buffer system.

$$Stability\ (\%) = (C_{Mn,\ total} - C_{Mn}(t)) /\ C_{Mn,\ total}$$

and

$$C_{Mn} = \frac{R_2 - R_{2,0} - r_{2,MnL} \times C_{Mn,total}}{r_{2,Mn} - r_{2,MnL}}$$

Figure 1 shows percent intact chelate over time measured by change in the relaxation rate T2.

**Example E - Complex stability (Plasma)**

Measurement of stability in human plasma. A buffered solution of Mn-chelate was added human plasma containing heparin as an anticoagulant. The concentration of the Mn-chelate was 100 μM Mn in the plasma sample. T2 relaxation rates were measured at 37°C using a Bruker mq60 minispec contrast agent analyzer. T2 relaxation rates of the mixture were periodically monitored. If test compounds are instable under these conditions, de-chelation during this test would lead to release $Mn^{2+}$, which binds to plasma proteins and/or low molecular weight species in the plasma sample. Proteinbinding of $Mn^{2+}$ influences the T2 relaxation time, this ongoing exchange reaction would lead to decreasing T2 relaxation times of the solution over time (measured at 1.41 T).

As described in Example D, the stability of the Mn chelate in plasma was determined based on the measurements of the relaxation of the Mn-chelate in plasma and calculated together with the known Mn concentration in the assay and the known relaxivity of unchelated $Mn^{2+}$ ($MnCl_2$) in plasma (r2 = 76 $mM^{-1}s^{-1}$) and the known relaxation rate of the pure plasma sample. Figure 2 shows percent intact chelate over time measured by change in the relaxation rate T2.

**Example F - Cell Toxicity (SH-SY5Y cells)**

Measurement of cell viability after exposure to different concentrations of the test item. Undifferentiated adherent SH-SY5Y cells were exposed to Mn-chelate at concentrations 1 and 5 mM for 24h in cell culture medium. Cell viability was measured using CellTiter Glo (Promega) following the manufacturers instructions. The luminescence was measured with a photometer (Spectramax, Molecular Devices). Viability is determined by comparing the luminescence recorded for Mn chelate and the reference compound (Gadovist, Bayer AG) in percent. Figure 3 shows the viablility percent of the Mn chelate.

**Example G - Partition coefficient (logP butanol/buffer)**

The partition coefficient P (log P= log($C_{butanol}/C_{buffer}$)) was determined in buffered aqueous solution (10 μM) of compound in 0.5 mL 50 mM Tris-HCl saturated with Butanol, pH 7.4) and 1-butanol 1+1 and the mixture was shaken for 2h at room temperature (n=3). The Mn-concentrations in each phase. $C_{butanol}$ and $C_{buffer}$ were measured by ICP-MS (The Chemistry of Contrast Agents in Medical Magnetic Resonance Imaging" 2nd Ed. Merbach AS, Helm L, Toth E, eds. Hoboken, NJ: Wiley 2013).

**Example H - NOAEL**

The no-observed-adverse-effect level (NOAEL) was tested in mice using a modified 4-level up procedure with

increasing doses of the test items. The observed NOAEL denotes the level of exposure, at which no severe adverse effects were observed (Dorato et al. Regul Toxicol Pharmacol. 2005 Aug;42(3):265-74). The test items were applicated as intravenous bolus injection via the tail vein. The starting dose was 0.5 mmol/kg bw (5-fold of the clinical dose of example 2), and the dose for the next level was increased to 1.0 mmol Mn/kg bw if the animals showed no adverse effects and decreased if the mice showed any adverse effects. The next level was 2.5 mmol Mn/kg bw. At the highest dose level of 5.0 mmol Gd/kg bw 3 animals were injected. Symptoms were observed continuously during the whole experiment (2h after contrast agent administration). Animals that survived were euthanized in deep anesthesia 7 days post injection (xylazine hydrochloride 20 mg/mL, Rompun 2%, Bayer Vital GmbH, Leverkusen and ketamine hydrochloride 100 mg/mL, Ketavet, Pfizer, Pharmacia GmbH, Berlin). Macroscopic analysis was performed in all mice immediately after death. Blood was collected by puncture of vena cava and ALT, AST, GGT, GLDH, creatinine and BUN were analyzed (300 μL serum).

**Example I - Plasma protein binding**

The binding of example 2 to plasma proteins of different species (human, dog, monkey, rabbit, mouse and rat) was investigated *in vitro* by using equilibrium dialysis in reusable 96-well Micro-Equilibrium Dialysis Devices (ht dialysis) (Banker MJ et al. J Pharm Sci. 2003 May;92(5):967-74).

**Example J - In vivo Pharmacokinetics Following i.v. Administration in Rat**

Pharmacokinetic parameters of Example 2 determined in male rats (Han-Wistar, n=3). The compounds were administered as a sterile aqueous solution into the tail vein as bolus of 100 μmol Mn/kg bodyweight. Plasma was sampled before and 1, 3, 5, 10, 15, 30, 60, 120, 240, 360 and 1440 min post administration and the Mn concentration was determined by Inductively Coupled Plasma Mass Spectrometry (ICP-MS Agilent 7500a, Waldbronn, Germany). The samples were digested using strong acidic and oxidizing conditions at elevated temperature following dilution in 1% nitric acid to bring the samples in the quantification range of the method. The lower limit of quantitation amounts to 1 nmol/L for all elements and the upper limit to 1000 nmol/L in the diluted sample.

The fit of the obtained data to a three-compartment model (Phoenix, WinNonlin 6.4, 3- compartment mode) yielded the pharmacokinetic parameters.

**Example K - Biliary elimination in bile duct cannulated (BDC) rats**

The biliary excretions of compounds were investigated in bile duct cannulated (BDC) rats (Burden N et al. Lab Anim. 2017 Oct; 51(5): 457-464). All animals are initially anesthetized using a body weight-adjusted intramuscular injection of a mixture (1+2) of xylazine hydrochloride (20 mg/mL, Rompun 2%, Bayer Vital GmbH, Leverkusen) and ketamine hydrochloride (100 mg/mL, Ketavet, Pfizer, Pharmacia GmbH, Berlin) using 1 mUkg body weight. The continuous anesthesia of the animals is achieved by the intravenous injection of 1.5 mL per hour (Braun perfusor infusion of 1+2, 1:20 saline, xylazine hydrochloride 20 mg/mL, Rompun 2%, Bayer Vital GmbH, Leverkusen and ketamine hydrochloride 100 mg/mL, Ketavet, Pfizer, Pharmacia GmbH, Berlin) via tail vein (Introcan, 24G, yellow). The animals received 0.1 mmol Mn/kg bw as intravenous bolus injection via tail vein (50 mmol Mn/L formulation). Bile was sampled 0-0.5h, 0.5-1h, 1-2h, 2-3h und 3-4h post injection. The manganese concentrations in the bile fractions were determined by Inductively Coupled Plasma Mass Spectrometry (ICP-MS Agilent 7500a, Waldbronn, Germany). The bile fractions (n=3 times 10 μL) were dried for 2h at 90°C and 50 μL 65% nitric acid ($HNO_3$, Fa. Fisher, Optima Grade #1219010) and 30 μL hydrogen peroxide 30% (H2O2 Fa VWR Chemicals, AnalaR NORMAPUR, #18J024022) were added and processed in microwave oven (5 cycles: 5min 40W, 5min 120W, 10min 200W, 10min 320W and 25min 400W, Fa. CEM, Mars5 Xpress). After the samples have cooled down to room temperature 920 μL of ICP diluent were added (1% HNO3 + 0.01% Triton Tx100 and internal standard 50 μM) Terbium), samples were adequately diluted, and the manganese concentrations were measured via ICP-MS.

**Example L - Excretion study in rats (5 days) and residual Manganese organ distribution (7 days)**

The excretion and organ distribution of the test item was determined in male rats (Han-Wistar, n=3). The compound was administered as a sterile aqueous solution as a bolus in the tail vein of the animals at an appropriate dose. Urine was collected in the following time periods 0-1h, 1-3h, 3-6h, 6-24h, 1-2d and 2-5d post injection and feces 0-1h, 1-3h, 3-6h, 6-24h, 1-2d and 2-5d post injection and feces 0-1d, 1-2d and 2-5d post injection. As a control, 3 animals were treated in the same way with saline. On day 7 the animals were sacrificed, and the following organs were excised: blood, liver, kidney, spleen, heart, lung, brain, muscle, skin, stomach, gut, bone and bone marrow. The remaining carcass was freeze dried and ground to a fine powder. The Mn concentration in the organs and the carcass was determined by

ICP-MS (ICP-MS Agilent 7500a, Waldbronn, Germany). The results of the organ distribution of investigated examples and saline control animals are summarized.

## Claims

1. A compound of general formula (I),

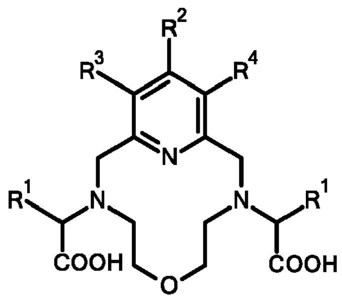

**(I)** ,

in which:

$R^1$ is a $-(CH_2)_m-(C{=}O)(NH)-(CH_2)-(CH(OH))_n-CH_2OH$ group
wherein m is an integer of from 1 to 3 and n is an integer of from 0 to 5;
$R^2$, $R^3$ and $R^4$ are each individually selected from a hydrogen atom, a $C_1$-$C_3$ alkyl group and a $C_1$-$C_3$ alkoxy group;
and stereoisomers, tautomers, N-oxides, hydrates, solvates, or salts thereof, or mixtures of same.

2. The compound according to claim 1, wherein

$R^1$ is a $-(CH_2)_m-(C{=}O)(NH)-(CH_2)-(CH(OH))_n-CH_2OH$ group
wherein m is an integer of from 1 to 2 and n is an integer of from 1 to 4;
$R^2$, $R^3$ and $R^4$ are each individually selected from a hydrogen atom, a $C_1$-$C_3$ alkyl group and a $C_1$-$C_3$ alkoxy group;
and stereoisomers, tautomers, N-oxides, hydrates, solvates, or salts thereof, or mixtures of same.

3. The compound according to any of claims 1 or 2, wherein:

$R^1$ is a $-(CH_2)_m-(C{=}O)(NH)-(CH_2)-(CH(OH))_n-CH_2OH$ group
wherein m is 2 and n is an integer of from 2 to 4;
$R^2$, $R^3$ and $R^4$ are each individually a hydrogen atom;
and stereoisomers, tautomers, N-oxides, hydrates, solvates, or salts thereof, or mixtures of same.

4. The compound according to any of claims 1 to 3,wherein:

$R^1$ is a $-(CH_2)_m-(C{=}O)(NH)-(CH_2)-(CH(OH))_n-CH_2OH$ group
wherein m is 2 and n is 4;
$R^2$, $R^3$ and $R^4$ are each individually a hydrogen atom;
and stereoisomers, tautomers, N-oxides, hydrates, solvates, or salts thereof, or mixtures of same.

5. The compound of general formula (I) according to any of claims 1 to 4 in the form of a complex with $Mn^{2+}$, and stereoisomers, tautomers, N-oxides, hydrates, solvates, or salts thereof, or mixtures of same.

6. A method of preparing a compound of general formula (I) in the form of a complex with $Mn^{2+}$ according to claim 5, said method comprising the step of allowing a compound of general formula (I)

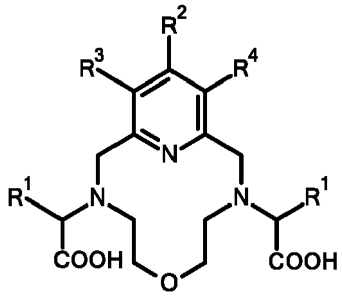

(I) ,

or a stereoisomer, tautomer, N-oxide, hydrate, solvate, or salt thereof, or mixtures of same in which $R^1$, $R^2$, $R^3$ and $R^4$ are as defined for the compounds of general formula (I), according to any one of claims 1 to 4, to react with a Manganese (II) salt,
thereby giving a compound of general formula (I), in which $R^1$, $R^2$, $R^3$ and $R^4$ are as defined for the compounds of general formula (I), according to any one of claims 1 to 4, in the form of a complex with $Mn^{2+}$.

**7.** Use of a compound of any one of claims 1 to 5 for diagnostic imaging, preferably for magnetic resonance imaging.

**8.** The compounds according to any one of claims 1 to 5 for use in diagnostic imaging, preferably for magnetic resonance imaging.

**9.** Use of the compounds according to any one of claims 1 to 5 or mixtures thereof for the manufacture of diagnostic agents, preferably for the manufacture of contrast agents for magnetic resonance imaging.

**10.** A method of imaging body tissue in a patient, comprising the steps of administering to the patient an effective amount of one or more compounds according to claim 5 in a pharmaceutically acceptable carrier, and subjecting the patient to magnetic resonance imaging.

**11.** Use of a compound of general formula (I)

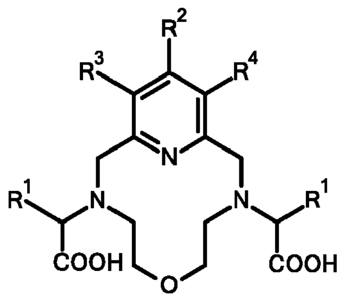

(I) ,

or a stereoisomer, tautomer, N-oxide, hydrate, solvate, or salt thereof, or mixtures of same in which $R^1$, $R^2$, $R^3$ and $R^4$ are as defined for the compounds of general formula (I), according to any one of claims 1 to 4, for the preparation of a compound of general formula (I) in the form of a complex with $Mn^{2+}$ according to claim 5.

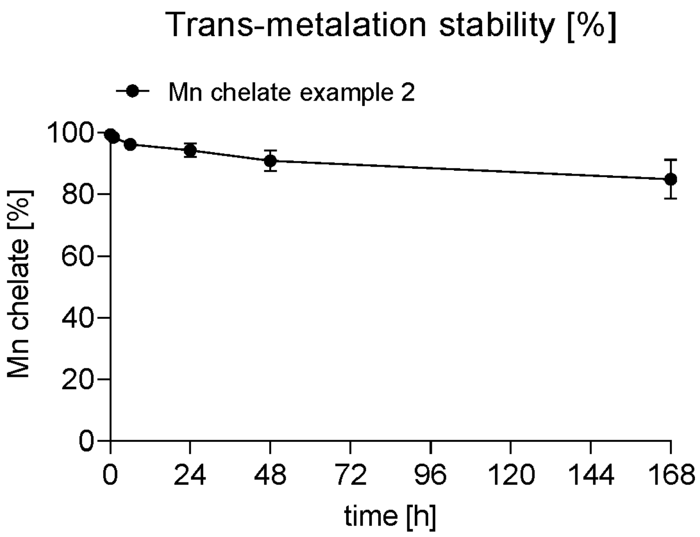


**Figure 1.**

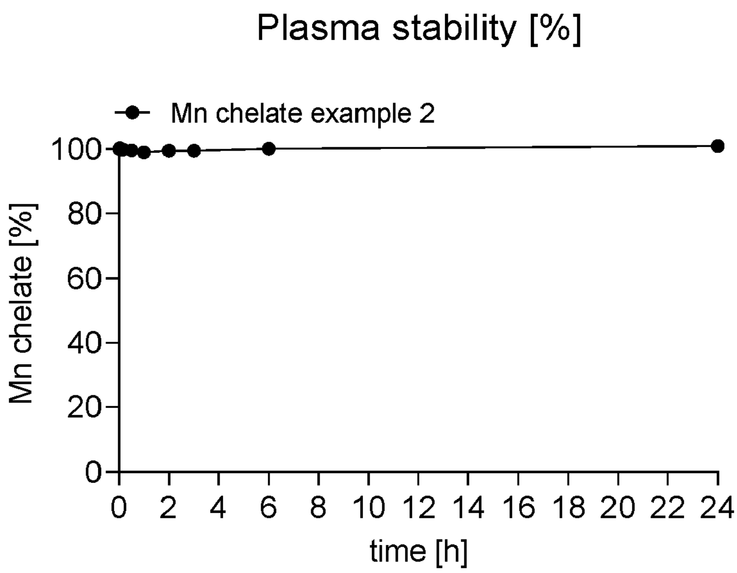


**Figure 2.**

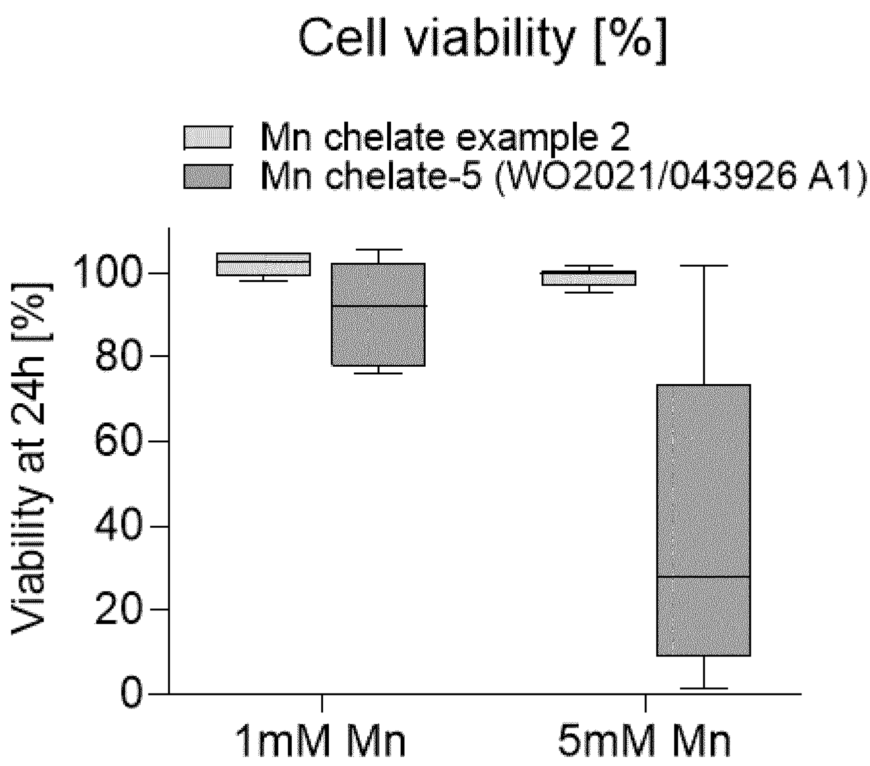
Cell viability [%]
Mn chelate example 2
Mn chelate-5 (WO2021/043926 A1)
Viability at 24h [%]
0
20
40
60
80
100
1mM Mn
5mM Mn

Figure 3.

Europäisches Patentamt
European Patent Office
Office européen des brevets

# EUROPEAN SEARCH REPORT

Application Number
EP 23 17 4013

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CSUPÁSZ TIBOR ET AL: "A New Oxygen Containing Pyclen-Type Ligand as a Manganese(II) Binder for MRI and 52Mn PET Applications: Equilibrium, Kinetic, Relaxometric, Structural and Radiochemical Studies", MOLECULES, vol. 27, no. 2, 7 January 2022 (2022-01-07), page 371, XP093094253, DE ISSN: 1433-1373, DOI: 10.3390/molecules27020371 * the whole document re 3,9-OPC2A, in particular the abstract, p.372-373, 387 and 388-390; figure 1; scheme 1; the tables * ----- | 1-11 | INV. C07D498/08 A61K49/10 C07F13/00 |
| Y | WO 2017/089849 A1 (DEBRECENI EGYETEM [HU]) 1 June 2017 (2017-06-01) * the whole document; in particular the claims; example 1; table 2 * ----- | 1-11 | |
| Y | WO 2017/220610 A1 (GE HEALTHACRE AS [NO]) 28 December 2017 (2017-12-28) * the whole document; in particular the claims; examples 1-3, 6, 8, 10; tables 4 and 5; figures such as figures 9-11 * ----- | 1-11 | TECHNICAL FIELDS SEARCHED (IPC): C07D |
| Y,D | WO 2021/043926 A1 (GE HEALTHCARE LTD [GB]) 11 March 2021 (2021-03-11) * the whole document; in particular the claims; examples 15-23; the figures * ----- | 1-11 | |

The present search report has been drawn up for all claims

1

| Place of search | Date of completion of the search | Examiner |
|---|---|---|
| Munich | 25 October 2023 | Hanisch, Inken |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 23 17 4013

This annex lists the patent family members relating to the patent documents cited in the above-mentioned European search report.
The members are as contained in the European Patent Office EDP file on
The European Patent Office is in no way liable for these particulars which are merely given for the purpose of information.

25-10-2023

| Patent document cited in search report | Publication date | Patent family member(s) | Publication date |
|---|---|---|---|
| WO 2017089849 A1 | 01-06-2017 | EP 3380484 A1 | 03-10-2018 |
| | | US 2018354969 A1 | 13-12-2018 |
| | | WO 2017089849 A1 | 01-06-2017 |
| WO 2017220610 A1 | 28-12-2017 | AU 2017281189 A1 | 17-01-2019 |
| | | BR 112018076389 A2 | 26-03-2019 |
| | | CA 3028027 A1 | 28-12-2017 |
| | | CN 109641900 A | 16-04-2019 |
| | | CN 111499633 A | 07-08-2020 |
| | | DK 3472163 T3 | 01-02-2021 |
| | | EP 3472163 A1 | 24-04-2019 |
| | | ES 2848580 T3 | 10-08-2021 |
| | | HU E053402 T2 | 28-06-2021 |
| | | JP 7048591 B2 | 05-04-2022 |
| | | JP 2019518082 A | 27-06-2019 |
| | | JP 2022101556 A | 06-07-2022 |
| | | KR 20190018710 A | 25-02-2019 |
| | | KR 20220123138 A | 05-09-2022 |
| | | LT 3472163 T | 25-05-2021 |
| | | PL 3472163 T3 | 31-05-2021 |
| | | RU 2018145061 A | 21-07-2020 |
| | | SG 11201811380R A | 30-01-2019 |
| | | US 2019233450 A1 | 01-08-2019 |
| | | WO 2017220610 A1 | 28-12-2017 |
| | | ZA 201900371 B | 30-10-2019 |
| WO 2021043926 A1 | 11-03-2021 | AU 2020340541 A1 | 21-04-2022 |
| | | BR 112022003242 A2 | 17-05-2022 |
| | | CA 3152191 A1 | 11-03-2021 |
| | | CN 114423761 A | 29-04-2022 |
| | | EP 4025259 A1 | 13-07-2022 |
| | | JP 2022546554 A | 04-11-2022 |
| | | KR 20220058574 A | 09-05-2022 |
| | | US 2022315616 A1 | 06-10-2022 |
| | | WO 2021043926 A1 | 11-03-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021043926 A1
- US 5560903 A
- WO 2017089849 A
- WO 2019122255 A
- EP 2457914 B1
- WO 2021043926 A


### Non-patent literature cited in the description


- **LAUFFER RB et al.** Paramagnetic metal complexes as water proton relaxation agents for NMR imaging: theory and design. *Chem Rev.,* 1987, vol. 87 (5), 901-27
- **CARAVAN P et al.** Gadolinium(III) chelates as MRI contrast agents: structure, dynamics, and applications. *Chem Rev.,* 1999, vol. 99 (9), 2293-352
- **FRENZEL et al.** *Invest Radiol.,* December 2008, vol. 43 (12), 817-28
- **KANDA et al.** *Radiology,* March 2014, vol. 270 (3), 834-41
- **TOFT KG et al.** Metabolism and pharmacokinetics of MnDPDP in man. *Acta Radiol,* July 1997, vol. 38 (4), 677-89
- **JIN et al.** Superparamagnetic iron oxide nanoparticles for MR imaging and therapy: design considerations and clinical applications. *Curr Opin Pharmacol.,* October 2014, vol. 18, 18-27
- **BOEHM-STURM et al.** Low-Molecular-Weight Iron Chelates May Be an Alternative to Gadolinium-based Contrast Agents for T1-weighted Contrast-enhanced MR Imaging. *Radiology,* February 2018, vol. 286 (2), 537
- **SNYDER et al.** A Class of FeIll Macrocyclic Complexes with Alcohol Donor Groups as Effective T1 MRI Contrast Agents. *Angew Chem Int Ed Engl.,* 03 February 2020, vol. 59 (6), 2414-2419
- *Pure Appl Chem,* 1976, vol. 45, 11-30
- **S. M. BERGE et al.** Pharmaceutical Salts. *J. Pharm. Sci.,* 1977, vol. 66, 1-19
- *CHEMICAL ABSTRACTS,* 3099-28-3
- **J. HENIG ; E. TOTH ; J. ENGELMANN ; S, GOTTSCHALK ; H. MAYER.** *H. Inorganic Chemistry,* 2010, vol. 49 (13), 6124-38
- **T.W. GREENE ; P.G.M. WUTS.** Protective Groups in Organic Synthesis. Wiley, 1999
- Database. 605005
- *CHEMICAL ABSTRACTS,* 2098905-67-8
- *CHEMICAL ABSTRACTS,* 488-43-7
- *CHEMICAL ABSTRACTS,* 25952-53-8
- *CHEMICAL ABSTRACTS,* 123333-53-9
- The Chemistry of Contrast Agents in Medical Magnetic Resonance Imaging. Wiley, 2013
- **DORATO et al.** *Regul Toxicol Pharmacol.,* August 2005, vol. 42 (3), 265-74
- **BANKER MJ et al.** *J Pharm Sci.,* May 2003, vol. 92 (5), 967-74
- **BURDEN N et al.** *Lab Anim.,* October 2017, vol. 51 (5), 457-464